# EUROPÄISCHE PATENTSCHRIFT

(11) **EP 1 093 783 B1**
(45) Veröffentlichungstag und Bekanntmachung des Hinweises auf die Patenterteilung: **03.12.2003**
(21) Anmeldenummer: 00113851.0
(22) Anmeldetag: 30.06.2000
(51) Int. Cl.: A61H 33/02, A61N 5/06, A61M 21/00, F21V 8/00

(54) **Wanne für die Hydro- und Chromotherapie**
Bath tub for Hydro- and Chromotherapy
Baignoire pour l'hydro- et chromothérapie

(30) Priorität: 21.10.1999 DE 29918615 U
(43) Veröffentlichungstag der Anmeldung: 25.04.2001
(73) Patentinhaber: Unbescheiden GmbH, 76532 Baden-Baden (DE)
(72) Erfinder: Unbescheiden, Veronika, 76547 Sinzheim (DE); Meissner, Markus, 73732 Esslingen (DE); Stoltz, Francis, 67100 Strasbourg (FR)
(74) Vertreter: WOLF & LUTZ

(56) Entgegenhaltungen:
- DE-A- 4 309 108
- FR-A- 2 701 842
- JP-A- 62 122 618
- US-A- 5 604 940

## Beschreibung

Die Erfindung betrifft eine Wanne für die Hydro- und Chromotherapie gemäß dem Oberbegriff des Patentanspruches 1.

Die Chromotherapie nimmt in der ganzheitlichen Medizin heute einen zunehmenden Stellenwert ein. Sie nimmt insbesondere Einfluß auf die Psyche des Menschen und dessen Hautreaktionen. Erfahrungswerte der ganzheitlichen Medizin belegen, daß sich physische Krankheiten und Beschwerden positiv durch die Psyche beeinflussen lassen. Es ist bekannt, durch Lichttherapie die Krankheiten oder Beschwerden zu heilen oder zumindest zu lindern.

Aus der US 5 604 940 ist eine Wanne bekannt, die eine Sprudeleinrichtung an ihrem Boden aufweist, durch die Luftblasen in das Badewasser einleitbar sind. Die Wanne weist außerdem eine Lichtquelle auf, deren Licht über Lichtleitfasern und einen Farbfilter in das Wanneninnnere einleitbar ist, um die Wanne dekorativ mit mehrfarbigem Licht zu beleuchten.

Aus der JP 62 122618 A ist eine Badewanne bekannt, in deren Inneres über eine Vielzahl von Lichtleitfasern Licht eingeleitet wird. Die Lichtleitfasern sind in Kunststoff eingebettet und emittieren an ihren Enden Licht in das Innere der Badewanne. Das Wasser in der Badewanne wird mit Licht aus jeder Richtung durchflutet, so dass das Licht von allen Seiten auf den Körper des Badenden einwirkt. Dabei kann das Licht in verschiedenen Farben und Farbtönen erzeugt werden.

In der DE 43 09 108 A1 ist eine Badewanne mit einem beleuchteten Innenraum beschrieben, bei der eine in der Wandung der Badewanne montierte Lichtquelle mit einer Glühlampe vorgesehen ist. Durch die vorbekannte Badewanne soll die Aufgabe gelöst werden, den beleuchteten Innenraum so zu gestalten, daß eine Montage der Lichtquelle erleichtert und an beliebigen Positionen möglich und eine Gefährdung der Badewanneninsassen durch den elektrischen Strom ausgeschlossen ist. Diese Aufgabe wird erfindungsgemäß dadurch gelöst, daß die Glühlampe räumlich außerhalb und im Abstand zur Wandung der Badewanne angeordnet ist und die Lichtquelle durch eine Lichtleitfaser optisch mit der Glühlampe verbunden ist.

In der FR-A-2 701 842 ist eine Wanne beschrieben, in der Lichtleitfasern in den Seitenwänden der Wanne enden, die zu einem Mehrfarbenprojektor führen, der eine Filterscheibe aufweist, in der bestimmte Farbfilter angeordnet sind. Dabei sind lediglich sieben verschiedene Farben vorgesehen. Außerdem weist die vorbekannte Anordnung nur insgesamt fünf Lichtleitfaserenden auf, wobei je zwei in den Seitenwänden und eine am Fußende angeordnet ist. Die Lichtleitfaserenden sollen zwar unter dem Niveau des Wassers in der Badewanne, jedoch oberhalb des Körpers des Patienten angeordnet sein. Damit kann aber keinesfalls eine vollständige Durchflutung des Wassers mit Licht aus jeder Richtung und eine Verteilung des Lichtes in der Wanne erfolgen.

Der vorliegenden Erfindung liegt nun die Aufgabe zugrunde, den Wirkungsgrad einer üblichen medizinischen Lichttherapie zu erhöhen und eine möglichst vollflächige Lichtbestrahlung der Hautflächen des Patienten zu erreichen.

Zur Lösung der gestellten Aufgabe wird eine Wanne gemäß Patentanspruch 1 vorgeschlagen, welche dadurch gekennzeichnet ist, dass zur Verteilung des Lichts in der Wanne und zur vollständigen Durchflutung des Wassers mit Licht aus jeder Richtung, und damit zur nahezu vollflächigen medizinisch wirksamen Lichtbestrahlung des Patienten, 150 bis 200 oder mehr Lichtleitfasern in der Wanneninnenfläche enden und dass die Luftsprudelbadeeinrichung eine Erhöhung der diffusen Reflexion und der diffusen Lichtbrechung des über die Lichtleitfasern eingebrachten Lichtes bewirkt.

Dadurch wird das Medium "Badewasser" als Lichtdiffusor genutzt. Die zusätzliche Einrichtung zur Erzeugung eines Sprudelbades bewirkt eine Erhöhung der diffusen Reflexion und der diffusen Lichtbrechung des über die Lichtleitfasern eingebrachten Lichtes. Kleine, umherwirbelnde und aufsteigende Luftbläschen spiegeln an den Grenzflächen das Licht unregelmäßig diffus innerhalb des Badewassers, so dass die Hautfläche des Patienten durch eine hohe Anzahl der Lichtquellen und des Wassermediums nahezu vollständig bestrahlt wird.

Gemäß Anspruch 2 kann der Mehrfarbenprojektor eine Farbauswahl von bis zu zweihundert Farben ermöglichen.

Vorzugsweise besteht die Einrichtung zur Erzeugung eines Sprudelbades aus einer Luftturbine mit Luftdüsen im Boden und/oder in den Wänden der Wanne.

Anhand der Zeichnung soll am Beispiel einer bevorzugten Ausführungsform die Wanne gemäß der Erfindung näher erläutert werden.

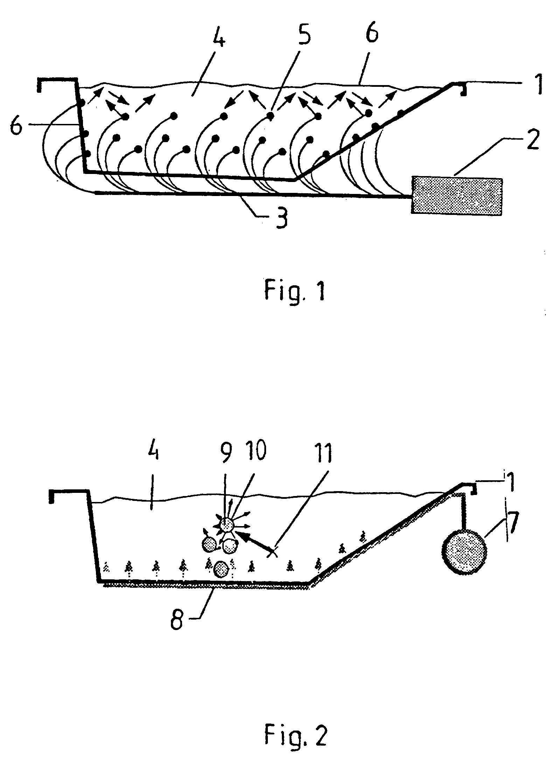

In der Zeichnung zeigt
**Fig. 1**
   einen prinzipiellen Schnitt durch die Wanne gemäß der Erfindung.
**Fig. 2**
   zeigt eine abgewandelte Ausführungsform der Wanne gemäß Fig. 1 mit Luftdruckdüsen.

Wie sich aus den Figuren der Zeichnung ergibt, besteht die prinzipiell dargestellte Wanne 1 aus einem beliebigen Kunststoff, beispielsweise aus Acrylglas. Über den Mehrfarblichtprojektor 2 und die Lichtleitfasern 3, die an allen Seitenwänden und dem Wannenboden mit dem Querschnitt der Fasern enden, wird das vom Mehrfarblichtprojektor 2 erzeugte Licht in der Wanne verteilt. Das Badewasser 4 wirkt als Lichtdiffusor, der das eintretende Licht 5 an den Grenzflächen des Badewassermediums 6 bricht, so daß das Wasser voll mit Licht aus jeder Richtung durchflutet und der Patient allseitig mit Licht bestrahlt wird.

Wie sich aus Fig. 2 ergibt, ist an der Wanne 1 eine zusätzliche Luftsprudelbadeeinrichtung 7 vorgesehen, die aus einer Luftturbine und aus Luftdüsen 8 am Boden der Wanne besteht. Dadurch entstehen innerhalb des Badewassers 4 Luftbläschen 9, an deren Luft-Wasser-Grenzflächen 10 sich das über die Lichtleitfasern eingebrachte Licht 11 unregelmäßig bricht, wodurch der Diffusionseffekt zusätzlich verstärkt wird.

Die Lichtbestrahlung kann im allgemeinen durch bis zu 200 Farben erfolgen.

Mit der Erfindung wird eine nahezu vollflächige Lichtbestrahlung des Patienten erreicht. Rotierende oder feststehende Filterscheiben innerhalb des Lichtprojektors ermöglichen einerseits durch den Therapeuten eine Farbauswahl bis zu 200 Farben und andererseits einen Farbwechsel während der Lichttherapie. Nach Vorauswahl aus einer Liste von 200 Farben kann der Therapeut zur Behandlung sechs oder mehr Lichtfarben auswählen.

## Patentansprüche

1. Wanne für die Hydro- und Chromotherapie, bestehend aus einem mit Wasser befüllbaren Wannenkörper (1) aus Kunststoff, wobei an mindestens einer Wannen-Innenfläche die Enden von Lichtleitfasern (3) angeordnet sind, die zu einem Mehrfarbenprojektor (2) führen, und wobei in der Wanne eine zusätzliche Luftsprudelbadeeinrichtung (7) angeordnet ist, ***dadurch gekennzeichnet*, dass** zur Verteilung des Lichtes in der Wanne und zur vollständigen Durchflutung des Wassers mit Licht aus jeder Richtung, und damit zur nahezu vollflächigen medizinisch wirksamen Lichtbestrahlung des Patienten, 150 bis 200 oder mehr Lichtleitfasern (3) in der Wanneninnenfläche enden, und dass die Luftsprudelbadeeinrichtung (7) eine Erhöhung der diffusen Reflexion und der diffusen Lichtbrechung des über die Lichtleitfasern eingebrachten Lichtes bewirkt.

2. Wanne nach Anspruch 1, ***dadurch gekennzeichnet,* dass** der Mehrfarbenprojektor (2) eine Farbauswahl bis zu zweihundert Farben ermöglicht.

3. Wanne nach einem der Ansprüche 1oder 2, **dadurch gekennzeichnet, dass** die Luftsprudelbadeeinrichtung (7) aus einer Luftturbine mit Luftdüsen im Boden und/oder in den Wänden der Wanne besteht.

## Claims

1. A bathtub for hydro- and chromotherapy, consisting of a bathtub body (1) of plastics fillable with water, the ends of optical fibres (3) being arranged at at least one inner surface of the bathtub, which optical fibres (3) lead to a multi-colour projector (2), and an additional air bubble-jet device (7) being arranged in the bathtub, ***characterised in that*,** to distribute the light in the tub and to flood the water completely with light from all directions, and thus to achieve virtually full-surface medically effective light irradiation of the patient, 150 to 200 or more optical fibres (3) end at the inner tub surface, and **in that** the air bubble-jet device (7) effects an increase in the diffuse reflection and diffuse refraction of the light introduced via the optical fibres.

2. A bathtub according to claim 1, ***characterised in that*** the multi-colour projector (2) allows a colour range of up to two hundred colours.

3. A bathtub according to one of claims 1 or 2, ***characterised in that*** the air bubble-jet device (7) consists of an air turbine with air nozzles in the bottom and/or in the walls of the tub.

## Revendications

1. Baignoire pour l'hydro- et la chromothérapie, constituée d'un corps de baignoire (1) en matière plastique, pouvant être rempli d'eau, dans lequel sur au moins une surface intérieure de la baignoire sont disposées les extrémités de fibres optiques (3), qui conduisent à un projecteur polychrome (2), et un dispositif de bain bouillonnant à bulle d'air (7) supplémentaire étant disposé dans la baignoire, **caractérisé en ce que**, pour répartir la lumière dans la baignoire et pour que la lumière venant de chaque direction traverse complètement l'eau, et ainsi pour qu'il se fasse une irradiation lumineuse, efficace d'un point de vue médical, sur presque toute la surface du patient, 150 à 200 fibres optiques (3) ou plus terminent dans la surface intérieure de la baignoire, et **en ce que** le dispositif de bain bouillonnant à bulle d'air (7) augmente la réflexion diffuse et la réfraction diffuse de la lumière amenée par les fibres optiques.

2. Baignoire selon la revendication 1, **caractérisée en ce que** le projecteur polychrome (2) permet de sélectionner jusqu'à deux cents couleurs.

3. Baignoire selon l'une des revendications 1 ou 2, **caractérisée en ce que** le dispositif de bain bouillonnant à bulle d'air (7) est constitué par une turbine à air comprenant des buses d'air dans le fond et/ou dans les parois de la baignoire.
